Europäisches Patentamt

European Patent Office

Office européen des brevets

(11) Publication number: **0 289 339 B1**

## EUROPEAN PATENT SPECIFICATION

(45) Date of publication of patent specification: **15.07.92**  (51) Int. Cl.5: **G01N  33/569**

(21) Application number: **88303913.3**

(22) Date of filing: **29.04.88**

The file contains technical information submitted
after the application was filed and not included in
this specification

(54) **Detection of antibodies to human immunodeficiency virus.**

(30) Priority: **01.05.87 US 44665**

(43) Date of publication of application:
**02.11.88 Bulletin  88/44**

(45) Publication of the grant of the patent:
**15.07.92 Bulletin  92/29**

(84) Designated Contracting States:
**DE FR GB IT**

(56) References cited:
**EP-A- 0 124 320          EP-A- 0 135 352**
**EP-A- 0 201 716          EP-A- 0 214 709**
**EP-A- 0 233 044          EP-A- 0 233 061**
**WO-A-86/06414**

(73) Proprietor: **CAMBRIDGE BIOSCIENCE CORPO-
RATION**
**365 Plantation Street**
**Worcester Massachusetts 01605(US)**

(72) Inventor: **Riggin, Charles H.**
**54 Laurelwood Drive**
**Hopedale, MA 01747(US)**
Inventor: **Marciani, Dante J.**
**48 School Street**
**Hopkinton, MA 01748(US)**

(74) Representative: **Sheard, Andrew Gregory et al
Kilburn & Strode 30, John Street
London WC1N 2DD(GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

Rank Xerox (UK) Business Services

**Description**

This invention relates to a latex agglutination assay for the determination of antibodies to human immunodeficiency virus (HIV) with HIV-specific antigen coated, hydroxylated microbeads.

Human immunodeficiency virus (HIV) has been shown to be the etiologic agent for acquired immune deficiency syndrome (AIDS) (Barre-Sinoussi et al., "Isolation of a T-lymphotropic retrovirus from a patient at risk for acquired immune deficiency syndrome (AIDS), " Science, 220:868-871 (1983) and Gallo et al., "Frequent detection and isolation of cytopathic retroviruses (HTLV-III) from patients with AIDS or at risk from AIDS," Science, 224:500-503 (1984)). An antibody response to HIV indicates exposure and infection. Current clinical assays for antibodies to HIV are viral based enzyme immunoassays (EIA). Viral lysate EIAs offer the advantages of high sensitivity but the disadvantage of high rates of false positives, of being slow, requiring several hours to complete a test, and the further disadvantage of requiring sophisticated instrumentation that is not available in all laboratories.

A latex agglutination assay based on purified HIV-specific antigen could offer the advantages of relatively high sensitivity, specificity, speed, and simplicity in situations where the time and technology required for EIA may not be available or appropriate. Latex agglutination is a technology which, unlike EIA, is a direct assay for specific antibodies. This test is based upon cross-linking antigen attached to microbeads with antibodies to form visible aggregates. Latex microbeads are negatively charged and antigens may bind to the microbeads by means of hydrophobic and ionic adsorption rather than by covalent attachment. Because of the fatal nature of HIV infection, it is important that any latex agglutination assay developed for determining the presence of antibodies to HIV in an individual at risk be very accurate.

EP-A-0214709 discloses methods of detecting antibodies to HTLV-III by an hemagglutionation assay using polystyrene latex beads coated with a 21mer which corresponds with a portion of p41, a part of p120, an envelope protein of HTLV-III.

The present invention relates to a latex agglutination assay for antibodies to human immunodeficiency virus (HIV) in a sample from a person suspected of being infected. The assay is performed by mixing the sample with HIV-specific antigen coated hydroxylated microbeads, evaluating whether agglutination occurs, and determining therefrom the presence of the antibodies to HIV in the sample.

The invention also relates to kits for employing the latex agglutination assay.

The invention may be illustrated, by way of example, with reference to the accompanying drawings, in which:

Figure 1 is a histogram showing the reactivity of positive sera by the latex agglutination test using hydroxylated microbeads, with the reactivity of positive sera plotted versus the number of samples tested; and

Figure 2 is a graph showing the thermal stability of a recombinant polypeptide antigen, CBre3, on hydroxylated latex microbeads.

This invention relates to a latex agglutination assay for determining the presence of antibodies to HIV. This assay is a direct, rapid, very sensitive, and specific alternative to EIA for antibody testing for HIV. This assay may be used in an emergency clinical setting where there would be a need for a rapid test, 5 minutes or less, for antibodies to the AIDS virus, and in areas lacking EIA instrumentation.

In this invention, the latex hydroxylated microbeads are coated with HIV-specific antigen. The antigens that may be used in this invention include any immuno-dominant and immuno-specific HIV antigens that have hydrophobic domains able to interact with the latex microbeads. As used herein, HIV antigen refers to polypeptides specific for HIV, such as those derived from the envelope gene of HIV. These HIV antigens may be derived by fractionating HIV, expression of HIV recombinant DNA clones and isolating and purifying selected antigens, or synthetic synthesis of HIV-specific polypeptides.

Preferably, the HIV antigen is obtained through genetic engineering techniques. A suitable recombinant HIV antigen that may be used in this invention in CBre3, derived from the gp120 and gp41 regions of the HIV envelope (env) gene. CBre3 (also identified as G71A) is described in Thorn et al., "An enzyme immunoassay using a novel recombinant polypeptide to detect human immunodeficiency virus," J. Clin. Microbiol. (in press 1987) and in United States patent application, Serial No. 825,597, filed February 3, 1986. The recombinant antigen is also on deposit at the American Type Culture Collection (ATCC), accession number 53455.

Other recombinant HIV antigens may be used in this invention, provided that the antigens exhibit extensive hydrophobic domains. Examples of such recombinant HIV antigens are described in Chang, et al., Bio/Technology, 3:905-909 (1985); Cabradilla et al. Bio/Technology, 4:128-133 (1985); and U.S. 4,629,783.

The latex microbeads used in this invention can be any suitable biologically inert microbeads. As used herein, the term "latex" is understood to mean an emulsion or other dispersion of natural or synthetic rubber particles. Suitable microbeads may include, although are not limited to, polyvinyl toluene, styrene-butadiene latex, styrene-divinyl benzene latex, acrylic latex, and polystyrene latex. The microbeads used in this invention preferably have an average diameter of 0.1 to 3.0 microns more preferably from 0.4 to 1.0 microns.

Hydroxylated microbeads are manufactured by co-polymerization of styrene and hydroxylated styrene monomers in an emulsion of surfactant micelles. Hydroxylated latex is also derived from conventional polystyrene beads by "aging" the beads or by incubation at elevated temperatures (van den Hul and Vanderhoff, "Inferences on the mechanism of emulsion polymerization of styrene from characterization of the polymer end-groups," Br. Polym. J., 2:121-127 (1970)). The sulfate groups present on the surface of polystyrene latex beads are hydrolyzed to hydroxyl groups.

Further, as used in this invention, the latex microbeads have a hydrophobic surface from which hydrophilic hydroxyl groups project. The inventors have discovered that using hydroxylated latex microbeads, in combination with the HIV specific antigen, results in increased activity or the ability of the HIV specific antigen to bind to the antibodies that are being detected. Without being limited in any way, it is believed that this is due to the following phenomena. The hydroxylation of the latex microbeads provides hydrophilic regions on the surface of hydrophobic beads. The HIV antigen is largely hydrophobic and these regions strongly adsorb to the hydrophobic surface of the microbeads. However, HIV antigen also has a few hydrophilic immunodominant regions and these portions interact weakly with the hydrophilic hydroxyl groups. The coating and attachment of the HIV antigen to the hydroxylated microbeads enhances the presentation of the antigen to antibodies and thereby the activity or ability of the antibody to bind to the antigen on one or more beads and subsequently cause measurable amounts of agglutination.

The hydroxylated latex beads may be prepared from HIV antigen coating by adaptation of known means for microbead coating. In the preferred embodiment of this invention, the hydroxylated latex beads are prepared for antigen coating by first suspending the beads in a buffer of pH from about 7 to about 10, preferably a sodium carbonate buffer, pH 9.6. Other buffers are known in the art and may be used in this invention, for example, borate-saline, glycine-saline, and N,N-bis-2-hydroxyethyl glycine. The HIV-antigen, dissolved in a buffer, typically a guanidine-HCl, Tris-HCl buffer, pH from about 7 to 9, is added to the latex bead suspension. Typically a 1 volume antigen to 2 volumes latex suspension will be sufficient to coat the latex beads with HIV-antigen. The latex bead suspension-HIV-antigen mixture is incubated for a time and under conditions sufficient to coat the beads. This is typically accomplished with several hours incubation time at room temperature. Other incubation times and temperatures can, however, be utilized. Next, bovine serum albumin (BSA) in buffered saline is used to block any uncoated portions of the beads. The HIV-antigen coated hydroxylated latex beads are then collected, typically by centrifugation.

The coated latex beads are suspended in a volume of 1% to 10% bovine serum albumin buffer to give a final antigen coated latex bead/buffer concentration of from about 0.4 to about 0.8% weight/volume. The patient samples (or laboratory samples) are also preferably diluted in BSA buffer. Positive control sample with antibody to HIV and negative control sample without virus-specific antibody should then also be diluted in BSA buffer.

The sample containing the antibodies to HIV will typically be a biological sample, although laboratory samples of antibodies may also be assayed. The biological sample may be blood plasma, serum, or whole blood from a patient. The sample may also include other biological fluids and tissue, such as, for example, urine, saliva, tear drops, cerebrospinal fluid, and the like, or a solid or semi-solid such as, for example, tissues, feces, and the like. The sample may be diluted to a range of from 1:0 parts to 1:100 parts sample:buffer (v/v) basis, with a BSA buffer as described above.

The agglutination assay of this invention is performed by mixing the sample suspected of containing antibodies to HIV with the HIV-antigen coated microbead suspension. The ratio of sample to microbead suspension may be any that gives an agglutination reaction for a positive sample. Those skilled in the art will be able to determine this ratio by routine experimentation using standards and controls. In a preferred embodiment of this invention, the ratio is from 5-25 ul microbead suspension and about 5 to 50 ul sample, more preferably 10 to 20 ul microbead suspension and about 15-40 ul sample; and most preferred 15 ul microbead suspension and 25 ul sample. Controls and standards are performed in like manner. The sample and microbead suspension mixture should be mixed for a time sufficient and under conditions to promote any agglutination. Typically, the sample and HIV antigen coated microbead suspension are mixed on a solid plate surface, such as a slide, and mixed by gentle rotation or shaking of the plate. The time required for sufficient agglutination is from about 3 to 5 minutes, typically about 3 minutes. A positive reaction for antibodies to HIV is agglutination visible in bright light. A negative reaction is no agglutination, the sample and microbead suspension mixture being a smooth consistency.

In addition, the materials for used in the assays of the invention are ideally suited for preparation of a kit. Such a kit may comprise a carrier means being compartmentalized to receive in close confinement one or more container means such vials, slides, and the like. Each of said container means comprises one of the separate elements to be used in the method.

For example, one of said container means may comprise HIV-antigen coated microbeads. A second container may comprise a buffer solution. A third and fourth container with negative and positive controls, respectively are included.

The carrier may also contain, in addition, a plurality of containers each of which comprises different, predetermined and known amounts of antibody to HIV and controls. These latter containers can then be used to prepare a standard curve from which can be interpolated the results obtained from the sample containing the unknown amount of antibody.

The following examples further describe the materials and methods used in carrying out the invention. The examples are not intended to limit the invention in any manner.

EXAMPLE I

Hydroxylated latex beads were prepared for antigen coating by first suspending the beads in sodium carbonate buffer, pH 9.6, collecting them by centrifugation, and then resuspending them in buffer three times more. The final suspension of hydroxylated beads was 5.0% weight/volume in carbonate buffer, pH 9.6; these were sonicated to ensure an even distribution. The HIV-antigen dissolved in 6M guanidine-HCl, 50 mM Tris-HCl buffer, pH 8.5, 0.5 mg/ml, was added dropwise to the latex suspension with constant mixing until the guanidine hydrochloride concentration in the reaction was 2.0 M (1 volume antigen to 2 volumes latex suspension). The mixture was incubated for several hours, with mixing, at room temperature. Next 1% bovine serum albumin in phosphate buffered saline was added until the reaction volume had been increased 5.5 fold. This suspension was incubated for 2 hours, with mixing at room temperature. The latex-antigen beads were collected by centrifugation, the supernatant was removed, and the beads resuspended in 1% bovine serum albumin in phosphate buffered saline, pH 7.6 (BSA buffer). The centrifugation and resuspension were repeated two times. The latex beads were collected by centrifugation, the supernatant removed, and the beads resuspended in a volume of BSA buffer equivalent to 16.67 times the original suspension volume for the adsorption step. This yielded a final latex-antigen concentration of 0.6% weight/volume. The latex-antigen suspension was sonicated to ensure an even distribution. The coated latex beads may be stored at 2-8°C.

EXAMPLE II

The data shown in Table 1 are from 50 individuals tested retrospectively by latex agglutination, Western blot, and EIA with CBre3 coated immunoassay plate. Four samples which were gag only by Western blot (data not shown) were positive by CBre3-latex agglutination even though no gag antigen was present on the latex. There was complete concordance between all samples tested by Western blot, CBre3-latex agglutination, and CBre3-EIA.

4

### Table I: Comparison of latex agglutination to Western blot of viral lysates and CBre3-EIA

| Total Tested | | | Western blot | | CBre3-EIA** | |
|---|---|---|---|---|---|---|
| | | | pos* | neg | pos | neg |
| 33 | pos | | 33 | 0 | 33 | 0 |
| | Latex Agglutination | | | | | |
| 17 | neg | | 0 | 17 | 0 | 17 |

50

*Includes 4 specimens which were gag only and 1 specimen which was p24 negative.

**Optical Density (O.D.) range 0.01 to 0.12 for negative samples and 0.61 to 2.0 for positive samples.

EXAMPLE III

A second set of 29 sera were tested retrospectively comparing latex agglutination, CBre3-EIA, and a commercially available viral lysate based EIA. The results are shown in Table II. There was 100% concordance between all samples tested. Even though the optical densities for some of the specimens by commercial EIA were as low as 0.517, latex agglutination and CBre3-EIA were still able to detect antibodies to HIV in these samples. In this group, samples for latex agglutination were tested both undiluted and diluted 1:10; this dilution did not affect whether a sample scored as positive or negative. However, some of the positives demonstrating high optical densities by EIA had a more intense reaction by agglutination at 1:10 than undiluted. This means that a prozone effect is likely with high titer seropositives.

**Table II:  Comparison of latex agglutination and CBre3-EIA**

**with recombinant _env_ to commercial EIA**

|  |  | Commercial EIA* | |
|---|---|---|---|
|  |  | + | – |
| Latex Agglutination | + | 24 | 0 |
|  | – | 0 | 5 |
| CBre-3-EIA** | + | 24 | 0 |
|  | – | 0 | 5 |

*O.D. range for positive samples was 0.517 to >2.0; for negative samples the range was .086 to 0.105 O.D.

**CBre3-EIA O.D. range for positive samples was 0.449 to >2.0; for negative samples the range was 0.102 to 0.162.

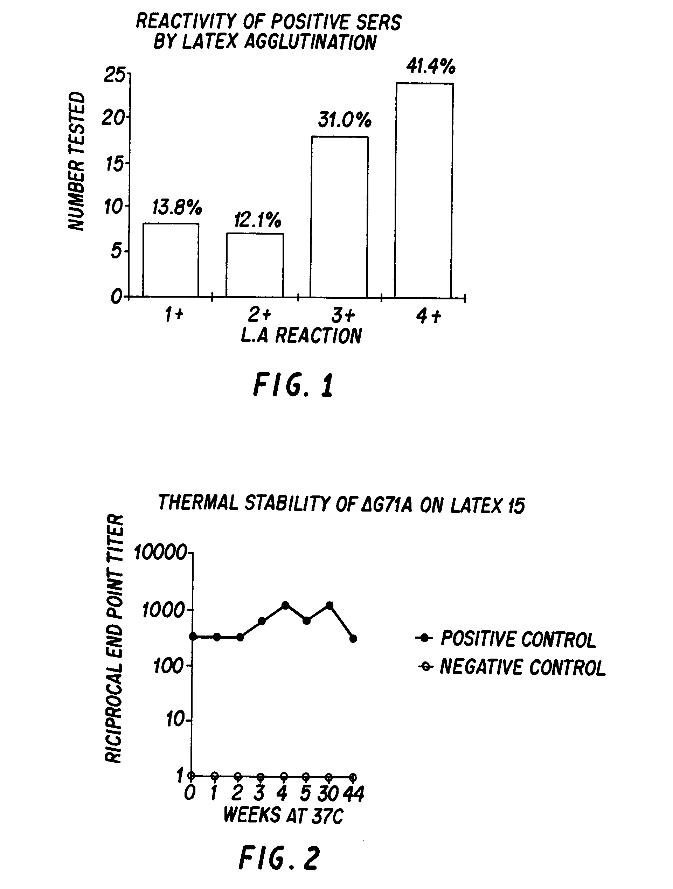

In Figure 1 are shown the relative intensity of the reactivities of the same 57 positive sera from Tables I and II by latex agglutination. A 4 + reaction is the strongest and most obvious reaction and a 1 + reaction is the least discernible above the lack of reactivity with a negative control. These data indicate that most of the 57 positive sera are strongly reactive by latex agglutination and are therefore easily read.

EXAMPLE IV

An experiment was performed to compare agglutination and CBre3-EIA with respect to end point dilutions of positive sera. Negative serum was used to dilute three different positive sera. Each diluted sample was then assayed by latex agglutination and CBre3-EIA. The results in Table III show similar reactivities by CBre3-EIA and latex agglutination with these three diluted positive sera. This means that latex agglutination with recombinant _env_ has sensitivities similar to EIA with these sera. It should be pointed out that the specimen can be tested undiluted by latex agglutination while EIA generally requires that the specimen by diluted at least 1:20 during the procedure.

**TABLE III.   Comparison of latex agglutination to CBre3-EIA with diluted sera.**

| Serum/ Dilution | 1:9 | 1:27 | 1:181 | 1:843 | 1:2400 | |
|---|---|---|---|---|---|---|
| 1 | 4+ | 4+ | 3+ | N | N | Latex Agglutination |
|   | 1.20 | 0.72 | 0.41 | 0.16 | 0.08 | O.D. by CBre3-EIA |
| 2 | 4+ | 4+ | 3+ | N | N | Latex Agglutination |
|   | 1.10 | 0.93 | 0.49 | 0.18 | 0.08 | O.D. by CBre3-EIA |
| 3 | 4+ | 3+ | N | N | N | Latex Agglutination |
|   | 0.70 | 0.47 | 0.16 | 0.08 | 0.02 | O.D. by CBre3-EIA |

**N is no agglutination or a negative reaction; O.D. 0.20 is a negative reaction by CBre3-EIA. Positive sera numbers 1, 2, and 3 were diluted with a negative serum to generate the dilutions indicated.**

EXAMPLE V

The thermal stability of CBre3 antigen on latex as determined by incubating a preparation at 37°C and testing aliquots at various times with positive and negative serum. Negative serum was diluted 1:10, positive sera was diluted serially from 1:10 until there was no agglutination. End point titer, the last dilution which had at 1+ agglutination, was plotted versus times at 37°C. The results of this experiment are shown in Figure 2. In Figure 2, the closed circles show endpoint titer by agglutination of a serum positive for antibodies to HIV versus weeks at 37°C for the latex-antigen; open circles show the same for negative serum. These results indicate CBre3-latex has reactivity with positive serum after 44 weeks at 37°C and no reactivity with negative serum during the same period.

**Claims**

1. A method for determining the presence of antibodies in a sample to human immunodeficiency virus (HIV) comprising contacting a sample suspected of containing antibodies to HIV with HIV-specific antigen coated, hydroxylated microbeads, determining whether agglutination occurs, and determining therefrom the presence of antibodies and optionally the amount of antibody that may be present in the sample.

2. A method as claimed in claim 1 wherein the HIV-specific antigen is an antigen from the envelope region of HIV containing hydrophobic domains.

3. A method as claimed in claim 1 or 2 wherein the HIV-specific antigen is a genetically engineered recombinant antigen.

4. A method as claimed in claim 3 wherein the recombinant HIV antigen is CBre3.

5. A method as claimed in any of claims 1 to 4 wherein the HIV-specific antigen is obtained by synthetic synthesis of HIV-specific polypeptides.

7

**6.** A method as claimed in any of claims 1 to 5 wherein the microbeads have an average diameter of from 0.1 to 3.0 microns.

**7.** A method as claimed in any of claims 1 to 6 wherein the sample is a biological sample.

**8.** A method as claimed in any of claims 1 to 7 wherein the sample is blood, blood plasma, serum, urine, saliva, tear drops, cerebrospinal fluid, tissue, faeces, sperm or vaginal fluids.

**9.** A method as claimed in any of claims 1 to 8 wherein the microbeads are suspended in a buffer solution prior to mixing with the sample.

**10.** A kit for determining the presence and optionally the amount of antibodies to human immunodeficiency virus (HIV) in a sample suspected of containing antibodies to HIV, the kit comprising;
HIV-specific antigen coated hydroxylated microbeads, and
means for contacting the sample with the microbeads to allow determination of any agglutination.

**Revendications**

**1.** Méthode pour déterminer la présence, dans un échantillon, d'anticorps dirigés contre le virus d'immunodéficience humain (HIV), consistant à mettre un échantillon suspecté de contenir des anticorps dirigés contre le HIV en contact avec des microsphères hydroxylées revêtues d'un antigène spécifique du HIV, à déterminer s'il se produit une agglutination, et à en déduire la présence d'anticorps et facultativement la quantité d'anticorps susceptible d'être présente dans l'échantillon.

**2.** Méthode selon la revendication 1, dans laquelle l'antigène spécifique du HIV est un antigène provenant de la zone formant enveloppe du HIV, contenant des domaines hydrophobes.

**3.** Méthode selon la revendication 1 ou 2, dans laquelle l'antigène spécifique du HIV est un antigène recombinant obtenu par génie génétique.

**4.** Méthode selon la revendication 3, dans laquelle l'antigène recombinant du HIV est CBre3.

**5.** Méthode selon l'une quelconque des revendications 1 à 4, dans laquelle l'antigène spécifique du HIV est obtenu par synthèse synthétique des polypeptides spécifiques du HIV.

**6.** Méthode selon l'une quelconque des revendications 1 à 5, dans laquelle les microsphères ont un diamètre moyen de 0,1 à 3 microns.

**7.** Méthode selon l'une quelconque des revendications 1 à 6, dans laquelle l'échantillon est un échantillon biologique.

**8.** Méthode selon l'une quelconque des revendications 1 à 7, dans laquelle l'échantillon est du sang, du plasma sanguin, du sérum, de l'urine, de la salive, des larmes, du fluide cérébro-spinal, du tissu, des fèces, du sperme ou des fluides vaginaux.

**9.** Méthode selon l'une quelconque des revendications 1 à 8, dans laquelle les microsphères sont mises en suspension dans une solution tampon avant mélange avec l'échantillon.

**10.** Kit pour déterminer la présence et facultativement la quantité d'anticorps dirigés contre le virus d'immunodéficience humain (HIV) dans un échantillon suspecté de contenir des anticorps dirigés contre le HIV, le kit comportant :
des microsphères hydroxylées revêtues d'un antigène spécifique du HIV, et
des moyens pour mettre l'échantillon en contact avec les microsphères, afin de permettre la détermination d'une quelconque agglutination.

**Patentansprüche**

**1.** Verfahren zur Bestimmung der Gegenwart von Antikörpern gegen den menschlichen Immunschwäche-Virus (human immunodeficiency virus, HIV) in einer Probe, umfassend das Inkontaktbringen einer Probe, die vermutlich HIV-Antikörper enthält, mit hydroxylierten Mikroperlen, die mit HIV-spezifischem Antigen beschichtet sind, Bestimmung ob Agglutination auftritt, und daraus Bestimmung der Gegenwart von Antikörpern und gegebenenfalls der Menge an Antikörper, der in der Probe vorliegen kann.

2. Verfahren nach Anspruch 1, wobei das HIV-spezifische Antigen ein Antigen aus der Hüllregion des HIV ist, die hydrophobe Domänen enthält.

3. Verfahren nach Anspruch 1 oder 2, wobei das HIV-spezifische Antigen ein gentechnologisch hergestelltes rekombinantes Antigen ist.

4. Verfahren nach Anspruch 3, wobei das rekombinante HIV-Antigen CBre3 ist.

5. Verfahren nach einem der Ansprüche 1 bis 4, wobei das HIV-spezifische Antigen durch synthetische Synthese von HIV-spezifischen Polypeptiden erhalten wird.

6. Verfahren nach einem der Ansprüche 1 bis 5, wobei die Mikroperlen einen durchschnittlichen Durchmesser von 0,1 bis 3,0 Mikron haben.

7. Verfahren nach einem der Ansprüche 1 bis 6, wobei die Probe eine biologische Probe ist.

8. Verfahren nach einem der Ansprüche 1 bis 7 wobei die Probe Blut, Blutplasma, Serum, Urin, Speichel, Tränen, Rückenmarksflüssigkeit, Gewebe, Päzes, Sperma oder vaginale Flüssigkeiten ist.

9. Verfahren nach einem der Ansprüche 1 bis 8, wobei die Mikroperlen vor dem Vermischen mit der Probe in einer Pufferlösung suspendiert werden.

10. Kit zur Bestimmung der Gegenwart und gegebenenfalls der Menge Von Antikörpern gegen menschliches Immunschwäche-Virus (human immunodeficiency virus, HIV) in einer Probe, die vermutlich HIV-Antikörper enthält, wobei der Kit umfaßt:
mit HIV-spezifischem Antigen beschichtete hydroxylierte Mikroperlen, und
Mittel zum Inkontaktbringen der Probe mit den Mikroperlen, um bestimmenm zu können, ob eine Agglutination auftritt.

REACTIVITY OF POSITIVE SERS
BY LATEX AGGLUTINATION

FIG. 1

THERMAL STABILITY OF ΔG71A ON LATEX 15

FIG. 2